# EUROPEAN PATENT SPECIFICATION

(11) **EP 4 228 513 B1**
(45) Date of publication and mention of the grant of the patent: **17.06.2026**
(21) Application number: 21801632.7
(22) Date of filing: 18.10.2021
(51) Int. Cl.: A61B 6/00, A61B 6/03, A61B 6/08, A61B 90/13, A61B 34/20

(54) **RADIOLOGICAL IMAGING DEVICE WITH IMPROVED FUNCTIONALITY**
RADIOLOGISCHE BILDGEBUNGSVORRICHTUNG MIT VERBESSERTER FUNKTIONALITÄT
DISPOSITIF D'IMAGERIE RADIOLOGIQUE À FONCTIONNALITÉ AMÉLIORÉE

(30) Priority: 19.10.2020 IT 202000024583; 19.10.2020 IT 202000024592; 19.10.2020 IT 202000024598; 19.10.2020 IT 202000024601; 19.10.2020 IT 202000024607; 19.10.2020 IT 202000024613
(43) Date of publication of application: 23.08.2023
(60) Divisional application: 26170094.2
(73) Proprietor: Imaginalis S.r.l., 50019 Sesto Fiorentino (Firenze) (IT)
(72) Inventor: MANETTI, Leonardo, 50019 Sesto Fiorentino (IT); FORTUNA, Damiano, 50019 Sesto Fiorentino (IT); LEONORI, Massimiliano, 50019 Sesto Fiorentino (IT)
(74) Representative: Celestino, Marco
(86) International application number: PCT/IB2021/059550
(87) International publication number: WO 2022/084819

(56) References cited:
- WO-A1-2020/161583
- US-A1- 2015 208 993
- US-A1- 2019 038 240

## Description

The present invention relates to a radiological imaging device with improved functionality of the type specified in the preamble of the first claim.

In particular, the invention relates to a device configured to be used in the medical/veterinary field to at least obtain radiological images (such as tomography) of at least a portion of the internal anatomy of a patient.

Known radiological imaging devices, regardless of the analysis performed (tomography, radiology or fluoroscopy) have the same basic structure.

This basic structure provides a bed on which the patient is arranged, a control station for the device; an O or C-shaped gantry, defining a cavity wherein the portion to be analysed and carrying out the radiological acquisition is inserted; and a support supporting the gantry and the bed and able to translate the bed and gantry between them.

The gantry provides an X-ray source; a detector that receives x-rays after they have passed through the bed and the patient.

In the cases of a radiological device for CT (Computed Axial Tomography), it has a rotation organ that rotates the source and detector around the patient allowing them to acquire at different angles and therefore to have a three-dimensional reconstruction of the patient.

Examples of these devices are reported in US20150208993A1, US20190038240A1, US2004125915A1, WO2014001834 and US20030072416. The known art described includes some important drawbacks.

In particular, known radiological imaging devices are particularly bulky and therefore of reduced portability.

Another drawback is that the radiological imaging devices currently in use require slow and inaccurate aiming. In fact, the aiming is performed manually by the operator who, using a camera close to the source, must understand when the source is centred with respect to the portion to be analysed.

An important drawback is also represented by the fact that the known radiological imaging devices allow the execution of a limited typology of acquisitions by requiring the purchase of several machines, each of which specific for a type of radiological imaging.

A not secondary drawback is represented by the fact that the known radiological imaging devices are constituted by numerous and complex apparatuses which make the devices particularly expensive (both in the purchase phase and in the maintenance phase) and above all difficult to manufacture and use.

In this situation, the technical task underlying the present invention is to devise a radiological imaging device capable of substantially obviating at least part of the aforementioned drawbacks.

Within the scope of said technical task, an important object of the invention is to obtain a radiological imaging device of reduced size and easy to transport.

Another object of the invention is to provide a radiological imaging device which is easy to use and which, in particular, allows precise and rapid centring with respect to the portion to be analysed which allows the execution of several types of acquisitions.

A not secondary object of the invention is to have an imaging device of low cost and above all easy to manufacture and use.

The technical task and the specified aims are achieved by a radiological imaging device as claimed in the annexed claim 1. Examples of preferred embodiment are described in the dependent claims.

The characteristics and advantages of the invention are clarified below by the detailed description of preferred embodiments of the invention, with reference to the accompanying figures, in which:
the **Fig. 1** shows, in scale, a radiological imaging device according to the invention;

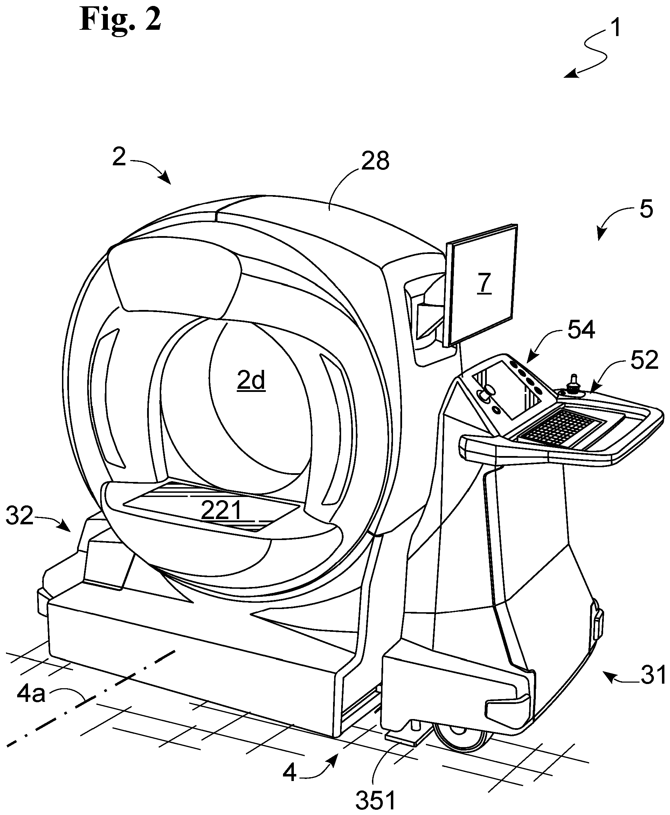

the **Fig. 2** illustrates, in scale, the device of Fig. in a different position;

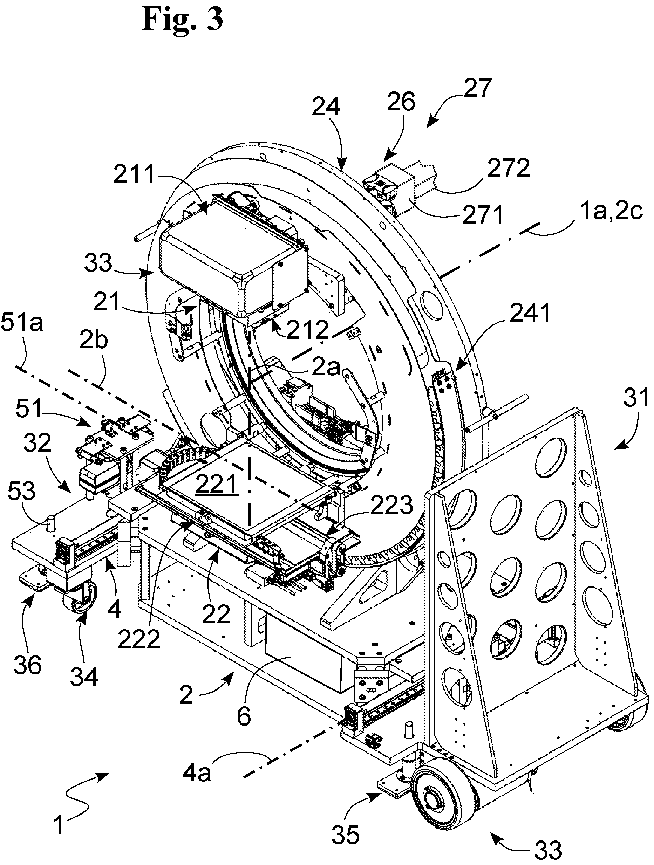

the **Fig. 3** shows, in scale, of an assembly of the radiological imaging device according to the invention;
the **Fig. 4** depicts, in scale, a second view of the assembly of Fig. 3;
the **Fig. 5** shows, in scale, of another radiological imaging device according to the invention;
the **Fig. 6** is a schematic of the operation of the radiological imaging device according to the invention; and
the **Fig. 7** shows a detail of the imaging device according to the invention.

In the present document, the measurements, values, shapes and geometric references (such as perpendicularity and parallelism), when associated with words like "about" or other similar terms such as "approximately" or "substantially", are to be considered as except for measurement errors or inaccuracies due to production and/or manufacturing errors, and, above all, except for a slight divergence from the value, measurements, shape, or geometric reference with which it is associated. For instance, these terms, if associated with a value, preferably indicate a divergence of not more than 10% of the value.

Moreover, when used, terms such as "first", "second", "higher", "lower", "main" and "secondary" do not necessarily identify an order, a priority of relationship or a relative position, but can simply be used to clearly distinguish between their different components.

The measurements and data reported in this text are to be considered, unless otherwise indicated, as performed in the International Standard Atmosphere ICAO (ISO 2533).

Unless otherwise specified, as results in the following discussions, terms such as "treatment", "computing", "determination", "calculation", or similar, refer to the action and/or processes of a computer or similar electronic calculation device that manipulates and/or transforms data represented as physical, such as electronic quantities of registers of a computer system and/or memories in, other data similarly represented as physical quantities within computer systems, registers or other storage, transmission or information displaying devices.

With reference to the Figures, the radiological imaging device according to the invention is globally indicated with the number 1.

It is configured to be used both in the medical and veterinary fields for the production and/or interpretation for diagnostic and/or therapeutic purposes of a radiological image (radiological imaging) of at least a portion of a patient to be analysed. In particular, the device 1 is configured to perform at least one tomography with suitably multiple stacks (or rather radiological (preferably tomographic) acquisitions of portions of the patient as described below).

It should be noted that the patient, during the acquisition, is on a radiological support (such as a radiological bed) defining a support surface for the patient and in particular for the portion to be analysed.

The radiological image is a representation of at least a portion to be analysed inside the patient.

The radiological image can include a sector of interest representative of the portion to be analysed suitably internal, optionally a perimeter sector of the sector of interest (for example identifiable in the patient's skin) and preferably a sector of non-interest that is not representative of the patient and visually separated from the sector of interest thanks to the perimeter sector.

In the case of 2D images, the sector of interest and that of non-interest can be surfaces/areas, while the perimeter sector can be represented by a line; while in the case of 3D images the sector of interest and that of non-interest can be volumes, while the perimeter sector can be represented by a surface.

The radiological imaging device 1 may comprise a radiological support.

The radiological support is known per se.

The radiological imaging device 1 is configured to rest on a support surface **1b** such as a walkable plane of a health facility. Conveniently it can be moved along said support surface 1b.

The radiological imaging device 1 can define a longitudinal axis **1a** suitably substantially not perpendicular and in detail substantially parallel to the supporting surface 1b when the radiological imaging device 1 is in use (hereinafter simply in use), or rather resting on said support surface 1b.

In this document terms such as "vertical" and "horizontal" define an axis, a displacement or other, respectively substantially perpendicular or substantially parallel to the supporting surface 1b when the radiological imaging device 1 is in use.

The radiological imaging device 1 can comprise a unit for controlling the operation of the device itself.

The control unit is configured to control and/or actuate, automatically and/or in response to a command given by the operator, the device 1 and in particular at least part and preferably at least one acquisition procedure **100** of radiological imaging feasible from the radiological imaging device 1 and described below.

The radiological imaging device 1 can comprise a gantry **2** configured to perform the radiological acquisition of at least a portion to be analysed.

The gantry 2 can be C-shaped (called C-arm) or preferably O-shaped (O-ring). Gantry 2 can define a scanning area **2d** wherein at least the portion to be analysed is available.

It can define a front face, a back face and two side faces.

In use, the faces are substantially transverse and in detail substantially perpendicular to the supporting surface 1b. The faces can identify the four lateral faces of an inscription cuboid of the gantry 2.

The rear and front faces can be substantially transverse and in detail substantially perpendicular to the longitudinal axis 1a.

The lateral faces can be substantially parallel to the longitudinal axis 1a.

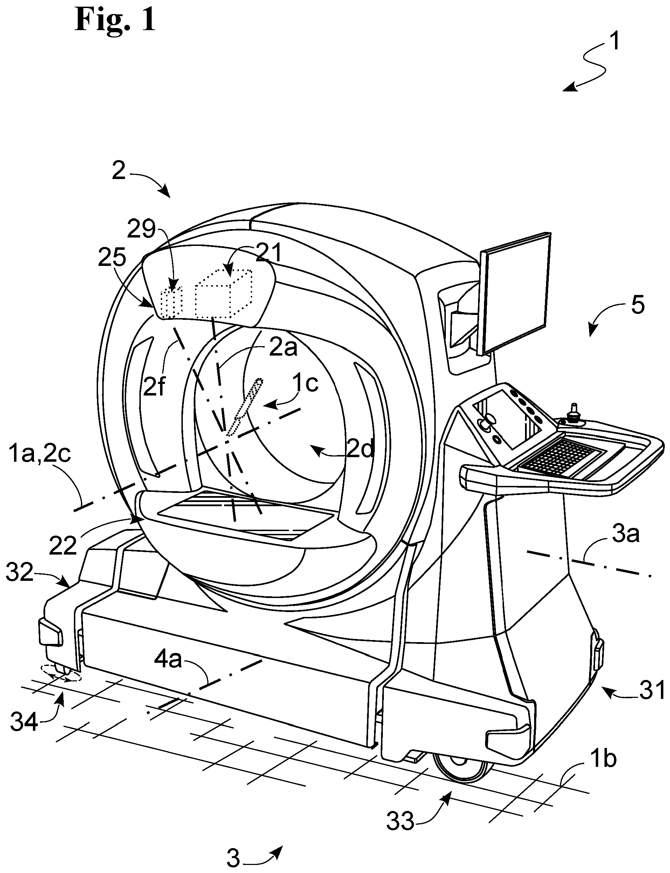

The gantry 2 can comprise a source **21** (schematically shown in Fig. 1) configured to emit and then define an acquisition beam and an acquisition axis **2a.**

Source 21 is in data connection and therefore can be controlled by the control unit. In use, the acquisition axis 2a can be substantially transverse to the support surface 1b.

The source 21 can emit an X-ray acquisition beam.

The source 21 can comprise an emitting body **211** of said acquisition beam defining the emission axis 2a; a collimator **212** configured to vary the section of the acquisition beam; and in some cases, a light source configured to illuminate the scanning area 2d and in particular the portion to be analysed, favouring the correct positioning of the acquisition beam.

Advantageously, the source 21 can be without a light source, as described in detail below.

It can be placed in correspondence with the front face of the gantry 2.

The source 21 can comprise a pointing apparatus configured to project a figure through which to perform the centring and therefore the pointing of the source with respect to the scanning area 2d and in particular to the portion to be to analyse. The gantry 2 can comprise a detector **22** configured to be etched by the acquisition beam after it has crossed the portion to be analysed.

The detector 22 configured to obtain at least one radiological acquisition when etched by said beam. Said at least one acquisition is then used by the control unit, in a known way, to obtain at least a radiological image.

The detector 22 defines a surface sensitive to the acquisition beam.

It is in data connection and therefore can be controlled from the control unit.

The sensitive surface can be substantially perpendicular to the acquisition axis 2a. The detector 22 can comprise a sensitive element **221** defining the sensitive surface.

The detector 22 can be placed in correspondence with the front face of the gantry 2.

The detector 22 can comprise a projector **222** configured to project an optical marker in the analysis area 2d, or rather on the support surface and in particular on the portion to be analysed so as to allow a precise positioning of the detector 22 and therefore of the source 21.

The optical marker can be a cross.

The projector 222 can comprise at least one light source (for example a laser source) defining said optical marker. In detail, it comprises two light sources suitably configured to emit two mutually incident optical emissions, defining said optical cross marker.

The projector 222 can be proximal to the sensitive surface. It can be integral with the sensitive element 221.

The detector 22 can comprise a waving device **223** configured to translate at least the sensitive element 221 along a waving axis **2b.**

The waving axis 2b can be substantially perpendicular to the acquisition axis 2a. The waving axis 2b can be substantially parallel to the sensitive surface.

The gantry 2 can comprise a support for source 21 and detector 22.

The support and therefore the gantry 2 can comprise a rotor **23** supporting at least source 21 and detector 22; and a stator **24** supporting the rotor 23 and configured to rotate the rotor 23 (and therefore the components constrained thereto) suitably defining a rotation axis **2c** and preferably according to the control unit.

The back and front faces of gantry 2 can be perpendicular to the axis of rotation 2c. The rotor 23 can define a scanning area 2d.

The source 21 and the detector 22 can be integral with the rotor 23.

They can be on the opposite side with respect to the acquisition zone 2d. Preferably, the stator 24 is configured to rotate the rotor 23 while keeping the acquisition axis 2a resting on a resting plane which, in use, can be practically transverse and in detail almost perpendicular to the support surface 1b.

The stator 24 comprises a rotation member **241** for the rotation of the rotor 23. The rotation axis 2c can be substantially parallel to the longitudinal axis 1a.

In use, the rotation axis 2c can be substantially non-perpendicular and in detail substantially parallel to the support surface 1b.

The rotation member 241 can be of a known type.

The rotation member 241 may comprise a rotation encoder configured to measure rotation about the rotation axis 2c.

It should be noted that the control unit can be at least in part and in detail totally constrained to the stator 24.

The gantry 2 can comprise an optical pointer **25** (schematically shown in Fig. 1) integral with the rotor 23 and configured to project an optical reference **2e** in the analysis area 2d, or rather on the support surface and in particular on the portion to be analysed.

The optical reference 2e can be a cross.

The pointer 25 is in data connection and therefore can be controlled by the control unit.

The optical pointer 25 is configured to project the optical reference 2e by defining a pointing axis **2f.**

The pointing axis 2f can be inclined with respect to the acquisition axis, suitably defining, with respect to the rotation axis 2c, a spreading angle therefore having a vertex on said rotation axis 2c.

The spreading angle can be substantially less than 180°, in detail at 90°, more in detail at 60° and more in detail still at 45°. It is preferably substantially comprised between 5° and 45° and more precisely between 10° and 30°.

The pointing 2f and acquisition 2a axes can be substantially coplanar and in detail resting on said resting plane.

They can be substantially incident the rotation axis 2c suitably in the same point.

The optical pointer 25 can comprise at least one emitter (suitably laser) defining said optical reference 2e. In detail, it comprises two emitters suitably configured to emit two mutually incident optical beams defining said cross-shaped optical reference 2e.

The optical pointer 25 can be angularly spaced from the detector 22 with respect to the rotation axis 2c by an angle at least equal to 120° and in detail substantially comprised between 130° and 160°. Consequently, the operator can check the correct positioning of source 21 and detector 22 with respect to the scanning area 2d and in particular to the portion to be analysed by using alternatively the projector 223 or the optical pointer 25.

The gantry 2 can comprise a connector **26** for an additional source **27.**

The connector 26 is configured to allow an additional source 27 to be associated with the radiological imaging device 1 which, therefore, is equipped with the sources 21 and 27.

The connector 26 is in data connection with the control unit which can therefore be placed in data connection with the additional source 27 and, for example, command its operation.

It is a quick fit type connector.

The connector 26 is of the resolvable type so as to allow to constrain or separate the additional source 27 from the gantry 2.

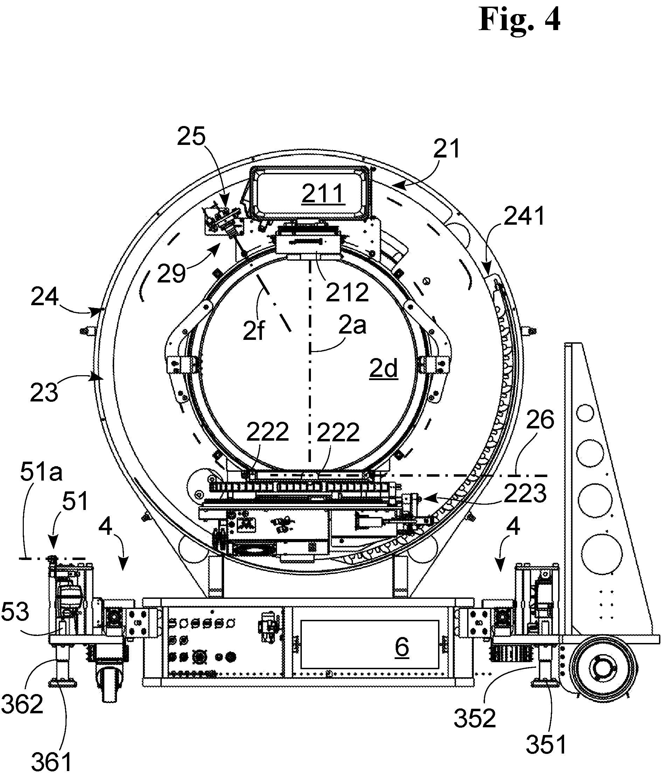

The connector 26 can be constrained on the opposite side with respect to the source 21 and detector 22 (Fig. 4) and in detail in correspondence with the face rear of the gantry 2.

The connector 26 can be integral with the support and in detail with the stator 24 or alternatively with the rotor 23.

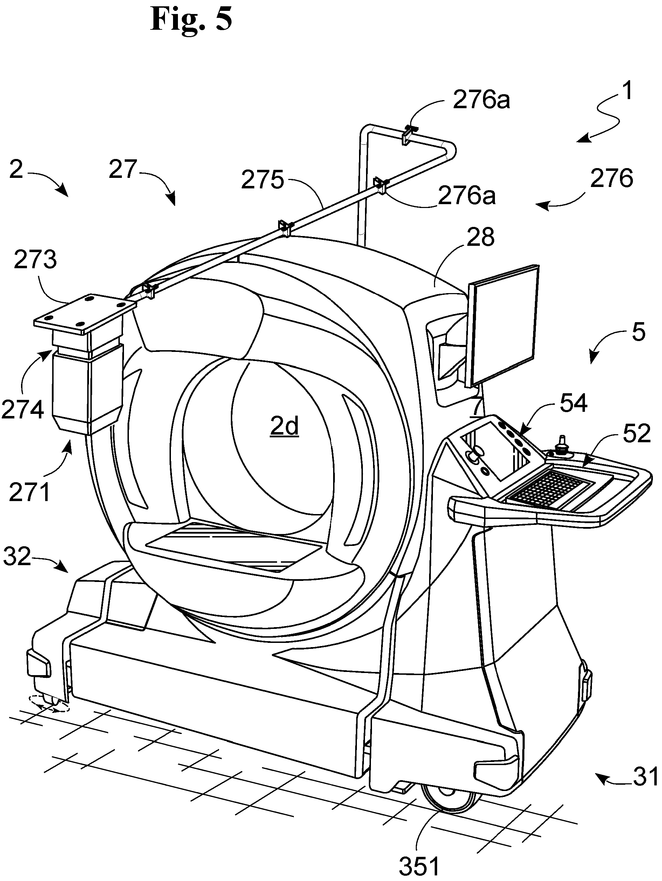

The gantry 2 can comprise the additional source 27 (Fig. 5).

The additional source 27 can be of the same type as the source 21. Alternatively, it can be of a different type so as to allow radiological acquisitions and therefore radiological images with different parameters and/or of a different type (for example magnetic).

The additional source 27 is configured to emit an additional acquisition beam configured to traverse a portion of the patient to be analysed and then be acquired by an additional detector of said additional beam.

The additional source 27 may comprise an additional emission apparatus **271.**

The additional apparatus 271 can comprise an additional body for emitting said additional acquisition beam, an additional collimator of the additional beam and in some cases an additional light source for illuminating the area 2d and in particular of the portion to be analysed.

The additional source 27 may comprise an additional connector **272** for engaging the connector 26.

The additional emission apparatus 271, when the additional source 27 is connected to the connector 26, may be close to the front face of the gantry 2 and, in particular, enclosing the source 21 between the additional apparatus 271 and the support (in detail the rotor 23).

The additional source 27 may not be supported by the gantry 2. It may comprise a support **273** configured to constrain the additional source 27 (in detail the additional emission apparatus 271) to an external structure (such as the supporting surface 1b a wall and/or a ceiling) so as to unload on it the weight of the additional source 27. The additional source 27 can comprise a driver **274** configured to move the additional emission apparatus 271 with respect to the support 273.

The driver 274 can be configured to translate the additional emission apparatus 271 with respect to support 273 along a vertical and/or horizontal direction.

The additional source 27 may comprise a connection cable **275** (suitably data and/or power supply) of the additional emission apparatus 271 to the additional connector 272; and suitably an additional support **276** configured to constrain the cable 275 to said external structure.

The additional support 276 can comprise one or more attachments **276a** configured to constrain solidly (preferably in a resolvable way) the cable 275 to said external structure.

It should be noted how the control unit, when the additional source 27 is associated with the connector 26, prevents the movement of the gantry 2 and in particular at least the rotation of the rotor 23.

The support 273 can comprise

The gantry 2 can comprise a casing **28** defining a housing for the above-described components of the gantry 2 except for the possible additional source 27.

It should be noted that the connector 26 is accessible from the outside of the casing 28.

The gantry 2 can comprise an acquirer **29** configured to perform an optical acquisition.

The acquirer 29 is configured to perform an optical acquisition along an axis substantially parallel to the positioning plane of the acquisition axis 2a in detail substantially parallel to the pointing axis 2f and in more detail substantially coinciding with the pointing axis 2f.

It is in data connection with the control unit.

The acquirer 29 is configured to perform an optical acquisition of at least part of the acquisition zone 2d and in particular of the portion to be analysed of a patient and therefore of at least part of the scanning zone 2d.

The acquirer 29 can be configured to perform an optical acquisition of at least the optical reference 2e.

It can be configured to perform an optical acquisition of at least one medical instrument **1c** so as to allow identification of the instrument and in particular the real position of the medical instrument 1c suitably with respect to the scanning area 2d and in particular to the portion to be analysed and for the precision to the optical reference 2e.

In particular, the radiological imaging device 1 and, to be more precise, the control unit can define the real position of the medical instrument 1c based on at least the acquisition of the optical reference 2e.

The term "position" in this document identifies one or more coordinates aimed at identifying the position of the medical instrument 1c with respect to a reference system, for example, the intersection between the rotation axis 2c (defining the X axis of said reference system) and resting plane (defining the Y and Z axes identifiable as perpendicular axes defined to each other on the basis of an angular rotor-stator position along said lying plane) of the gantry 2 at an end stroke along the axis 4a.

The optical reference 2e can identify the origin of a reference system with respect to which to position the medical instrument 1c.

For example, the optical reference 2e can identify a point where the operator places/arranges the medical instrument 1c. It can for example identify the insertion point of the medical instrument 1c. Therefore, the optical reference 2e can identify a point of the medical instrument 1c such as the functional end **1d** of the instrument, that is, from the end of the instrument to be used on the patient such as the cutting profile of a scalpel.

In addition or as an alternative, said real position/orientation of the medical instrument 1c can be defined on the basis of at least the acquisition of the same medical instrument 1c.

In particular, the acquirer 29 is configured to perform an optical acquisition of the medical instrument 1c through which the control unit can define the real position of the medical instrument 1c.

In some cases the device 1 and in detail the control unit can comprise an instrument database associating to each instrument a profile (suitably three-dimensional) of the medical instrument 1c and in particular of the functional end 1d so as to allow to verify the correct positioning of the medical instrument 1c and therefore of the functional end 1d with respect to the scanning area 2d and in particular to the portion to be analysed and, for accuracy, to the optical reference 2e.

The acquirer 29 can be integral with the rotor 23. It can therefore be rotated around the rotation axis 2c so as to perform an optical acquisition of at least part and in detail of the totality of the portion to be analysed.

The radiological imaging device 1 can comprise a support structure **3** for supporting the gantry 2 and resting, suitably in use, on the support surface 1b.

In use, the supporting structure 3 is configured to arrange the gantry 2 distant from the supporting surface 1b. In detail, the support structure 3 can define a vertical distance between the gantry 2 and the support surface 1b which is practically less than 30 cm, in detail at 20 cm and suitably substantially comprised between 1 cm and 10 cm.

The support structure 3 can comprise a first column **31** constrained to the gantry 2 suitably at a first lateral face of the gantry 2 and a second column **32** constrained to the gantry 2 suitably in correspondence with a second lateral face of the gantry 2 suitably opposite the first face with respect to the gantry 2.

The gantry 2 has a longitudinal length (or rather along the longitudinal axis 1a) almost at least equal and in detail almost superior to that of the support structure 3 and therefore of the columns 31 and 33.

The radiological imaging device 1 can be mobile and therefore the support structure 3 can comprise means for moving the radiological imaging device 1 on the support surface 1b.

The handling means can be in data connection and therefore can be controlled by the control unit.

The movement means can define a movement axis **3a** of the device 1 substantially perpendicular to the longitudinal axis 1a.

In detail, they comprise first driving means **33** associated with the first column 31 and second driving means **34** associated with the second column 32.

The first driving means 33 can be motorized and in detail comprise at least one driving wheel suitably defining a traction axis substantially parallel to the longitudinal axis 1a.

Additionally, the first means 33 can comprise at least one idle rolling element.

The second movement means 34 can be idle and in detail comprise at least one idle wheel suitably pivoting.

In order to stabilize the radiological imaging device 1 during a radiological acquisition, the supporting structure 3 can comprise at least one stop configured to lock the device 1 to the supporting surface 1b.

The at least one stop can define, for the radiological imaging device 1, a transport configuration wherein the driving means identify the only contact of the device 1 with the supporting surface 1b and a non-transport configuration wherein the driving means do not identify the only contact of the device 1 with the surface 1b and in detail are not in contact with the support surface 1b.

The at least one stop can comprise at least a first stop **35,** suitably only one, integral with the first column 31 and at least a second stop **36,** suitably only one, integral with the second column 32.

A first stop 35 comprises a first plug **351** configured to come into contact with the support surface 1b and first an actuator **352** configured to move the first plug 351 almost perpendicular to the support surface 1b. In the transport configuration, the first plug 351 is in contact with the support surface 1b; in the non-transport configuration, the first plug 351 is not in contact with the support surface 1b.

The first element 351 can comprise rubber or other high friction contact element with the support surface 1b.

A second stop 36 comprises a second plug **361** configured to contact the support surface 1b and a second actuator **362** configured to move the first plug almost perpendicular to the support surface 1b. In the transport configuration the second plug 361 is in contact with the support surface 1b; in the non-transport configuration the second plug 361 is not in contact with the support surface 1b.

The second element 361 can comprise rubber or other high friction contact element with the bearing surface 1b.

The radiological imaging device 1 can comprise a conduction apparatus **4** configured to control the movement of the device 1 along the supporting surface 1b and suitably therefore the driving means 33 and 34.

The radiological imaging device 1 can comprise at least one guide **4** defining a translation axis **4a** of the gantry 2 with respect to the support structure 3 suitably substantially parallel to the rotation axis 2c.

The translation axis 4a can be substantially parallel to the longitudinal axis 1a. It can be substantially parallel to the supporting surface 1b.

The translation axis 4a can be substantially parallel to the rotation axis 2c.

The translation axis 4a can be substantially perpendicular to the scanning axis and in detail to the waving axis 2b.

The guide 4 can be external to the vertical projection of the scanning zone 2d defining an access zone to the free zone 2d and therefore through which the patient can position himself in proximity to it, allowing the gantry 2 to translate along the translation axis 4a by performing a multi-stack radiological acquisition. In particular, it can be external to the vertical projection of the gantry 2.

The guide 4 can be external to the horizontal projection of the entire gantry 2.

Terms such as vertical and horizontal, as mentioned above, are to be understood as identifying a direction/axis/displacement substantially perpendicular and horizontal with respect to the support surface 1b when the device 1 rests thereon. The gantry 2 has a longitudinal length substantially at least equal to and in detail substantially greater than that of the guide 4 and therefore of the stroke of the guide 4.

The at least one guide 4 can comprise a first guide 4 interposed between the first column 31 and gantry 2 and therefore binding said first column 31 to said gantry 2 and a second guide 4 interposed between second column 32 and gantry 2 and therefore binding said second column 32 to said gantry 2.

Preferably both guides 4 are motorized.

They can be implemented synchronously.

The guides 4 can be in data connection and therefore can be controlled by the control unit.

At least one guide 4 can comprise a translation encoder configured to measure the translation along the translation axis 4a.

Optionally, the control unit, when the additional source 27 is associated with the connector 26, prevents the movement of the gantry 2 along the translation axis 4a. The radiological imaging device 1 can comprise a conduction apparatus **5** configured to control the movement of the device 1 along the resting surface 1b. The conduction apparatus 5 can be in data connection with the control unit.

It can comprise a shooting block **51** defining a shooting axis **51a** which is suitably almost transverse and in detail almost perpendicular to the longitudinal axis 1a. The shooting axis 51a can be substantially horizontal.

The shooting block 51 can comprise a camera suitably of the optical type.

It can be integral with the second column 32.

The conduction apparatus 5 can comprise control means **52.**

The control means 52 are configured to allow to control the movement of the imaging device 1 on the supporting surface 1b. Preferably they allow manual control and therefore can comprise at least one handle.

In addition and/or as an alternative, they allow automatic guidance of the device, that is, it can be controlled exclusively by the control unit which can be equipped, for example, with a geo-localization system.

The control means 52 can be integral with the first column 31.

It can be seen how the control means 52 can allow the operator to control the operation of the entire radiological imaging device 1 and therefore the execution of a radiological acquisition.

The conduction apparatus 5 may comprise a detector **53** of obstacles.

The detector 53 is configured to detect the presence of obstacles during any movement of the radiological imaging device 1.

In detail, it can detect the presence of obstacles during the movement of the device 1 along the supporting surface 1b. It can be integral with the first column 31 and/or with the second column 32.

Alternatively or in addition, the detector 53 can detect the presence of obstacles during the translation of the gantry 2 along the translation axis 4a. It can be integral with gantry 2.

It can comprise a sensor configured to emit a wave (such as ultrasonic and/or electromagnetic) configured to intercept an obstacle. Said sensor can be a known parking sensor.

The conduction apparatus 5 can comprise at least one screen **54** for viewing the filming of the shooting block 51 and/or of the detector 53.

The screen 54 can be integral with the first column 31.

The radiological imaging device 1 can comprise a power supply **6** of the entire radiological imaging device 1.

In particular, the power supply 6 can be connected to the connector 26 so as to power the possible additional source 27.

It can be at least partially and in detail totally constrained to the stator 24.

The power supply 6 can comprise at least one battery.

It may include a connection to an external power supply network.

The radiological imaging device 1 can comprise an interface 7 of data exchange between the operator and the control unit (and therefore the various components of the device).

The interface 7 can be integral with the gantry 2 and in detail with the stator 24. The interface 7 can be an input and therefore allow the operator to enter data such as, for example, the command data of device 1.

In addition or alternatively, the interface 7 can be an output and therefore can allow the device 1 to communicate data to the operator, such as, for example, the result of a radiological acquisition, that is, a radiological image.

It should be noted that the radiological imaging device 1 may have no patient support structure (such as a radiological bed) which, in any case, can be used in combination with the device 1 during a radiological acquisition. It is therefore structurally separated from the patient support structure and therefore movable (integrally or only the gantry 2) with respect to the patient support structure.

In some cases the radiological imaging device 1 and in detail the control unit can comprise an acquisitions database associated with each radiological acquisition and the point/angle (or rather the coordinates) wherein said radiological acquisition was performed. In particular, the acquisitions database allows to associate to each point of the radiological image (suitably constructed on the basis of one or more acquisitions) at least one virtual coordinate identifying the position of said point in the image, or rather. with respect to the scanning area 2d and in particular to the portion to be analysed. Said association

Said at least one virtual coordinate, can comprise the angular position (or rather the angle) of the rotor 23 with respect to the stator 24.

Finally, the device 1 can comprise one or more medical instruments.

It should be noted that the radiological imaging device 1 can comprise for one or more and preferably the totality of the movements (rotations and/or translations) performed/implemented by it, encoders or other measuring means of said movement so as to allow to associate to each point of the radiological image to a point in space relative to the same device. The device 1 and in detail the control unit are configured to associate, conveniently thanks to the acquisition database and/or said measurement means, to each virtual coordinate of a point in the radiological image a real coordinate (with respect to an absolute reference and/or of the device) able to allow to identify the position of the point with respect to the device.

The operation of the radiological imaging device 1 previously described in structural terms is as follows.

This operation defines a new acquisition procedure **100** which radiological imaging can be implemented by the radiological imaging device 1 and described below.

The radiological imaging acquisition procedure 100 is controllable and therefore executable by the control unit preferably automatically and/or in response to at least one command given by the operator.

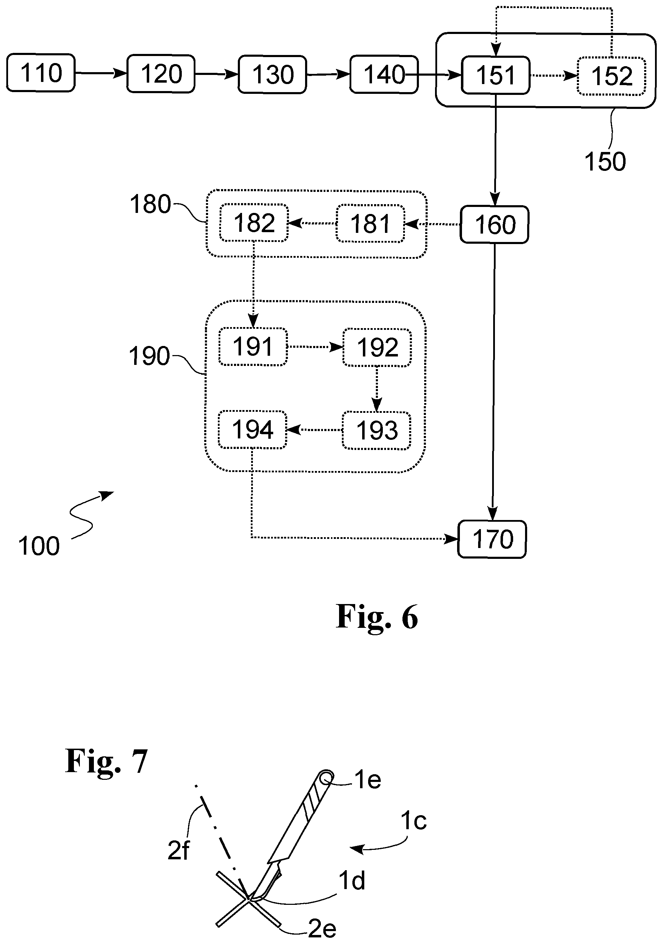

The acquisition procedure 100 is schematized in Fig. 6.

The acquisition procedure 100 can be implemented once the portion to be analysed or at least part of it is in the analysis area 2d.

The acquisition procedure 100 may comprise a placement phase **110** the device 1.

In this phase 110 the radiological imaging device 1, with the stops 35 and 36 in the transport configuration, is conducted in correspondence with the environment where the acquisition thanks to the driving means 33 and 34 and suitably to the conduction apparatus 5.

This operation can be performed automatically (therefore controlled by the control unit) and/or manually (therefore controlled by the operator).

Once the desired position has been reached, the placement phase 110 is concluded by bringing the radiological imaging device 1 into the non-transport configuration, or rather the stops 35 and 36 in contact, preferably exclusively, with the supporting surface 1b.

The acquisition method 100 can comprise a selection phase **120** of the radiological acquisition parameters.

Said phase can be controlled by the operator through said interface.

The acquisition method 100 can comprise a centring phase **130** of the device 1 with respect to the scanning area 2d and in particular to the portion to be analysed.

This operation can be performed using, for example, at least one of the pointing apparatus of the source 21, projector 222 of the detector 22 and radiological acquisitions, for example lateral, suitably of the fluoroscopy type.

In some cases the centring step 130 can be performed by exploiting the optical reference 2e with respect to the scanning area 2d and in particular to the portion to be analysed. In this case the source 21, detector 22 and optical pointer 25 are moved (suitably translated along the translation axis 4a and/or rotated around the translation axis 4a and/or they can be rotated with respect to the translation axis 2c) so to place the optical reference 2e in correspondence with the scanning area 2d in particular of the portion to be analysed. In this case, the acquisition method 100 can comprise a rotation phase **140** wherein the rotor 23 rotates by an angle equal to the angle of spread between the acquisition axis 2a and the pointing axis 2f, arranging the acquisition axis 2a substantially parallel to the pointing axis 2f in the centring phase 130. In detail, at the end of phase 140 the acquisition axis 2a is substantially in the position of the pointing axis 2f at the end of the centring phase 130.

After centring, the acquisition method 100 may comprise an acquisition phase **150** of at least a radiological image.

The acquisition phase 150 can comprise at least one scanning sub-phase **151** in which the source 21 emits an acquisition beam which crosses the portion to be analysed and is therefore detected by the detector 22 obtaining a radiological acquisition.

In the acquisition sub-step 151 the rotor 23 can rotate around the scanning area 2d suitably by an acquisition angle so as to simultaneously move source 21 and detector 22 around the portion to be analysed.

Conveniently, in each acquisition sub-phase 151 the above-mentioned database of acquisitions can be defined associating to each acquisition the point/angle wherein said radiological acquisition was performed.

The number of scanning sub-phases 151, or rather of radiological acquisitions, identifies the stack number. It is proportional to the ratio between the scanning length (or rather. the length of the portion to be analysed along the translation axis 4a) and the active length (or rather the length of the sensitive surface of the detector 22 along the translation axis 4a) suitably rounded up.

The active length can be stored on the control unit.

The acquisition length can be defined by the operator through this interface.

In detail, the acquisition phase 150 can comprise only one scanning sub-step 151 if the active length is substantially not greater and in detail substantially less than the acquisition length. In this case, device 1 performs a single stack acquisition.

Alternatively, the acquisition phase 150 can comprise a plurality of scanning sub-phases 151 if the active length is substantially greater than the acquisition length. In this case, device 1 performs a multi-stack radiological acquisition. In this case the phase 150 can suitably at least one translation sub-phase **152** wherein the at least one guide 4 translates the gantry 2 along the translation axis 4a.

This translation of the gantry 2 can be almost less than the active length.

Each translation sub-phase 152 is interposed between two consecutive scanning sub-phases 151.

It is highlighted that in the acquisition phase 150 and in detail in each scanning sub-phase 151 an optical acquisition of at least part and in detail of the totality of the portion to be analysed. In particular, the acquirer 29 can rotate around the rotation axis 2c by carrying out said optical acquisition.

At the conclusion of the acquisition phase 150, the acquisition process 100 can comprise a reconstruction phase **160** wherein the radiological image is reconstructed on the basis of one or more radiological acquisitions performed in the acquisition phase 150; and suitably a video displaying phase **170** for said radiological image via said interface.

Said phases 160 and 170 are known per se.

The acquisition method 100 can comprise a planning phase **180** in which an ideal position of a medical instrument 1c is suitably defined with respect to the scanning area 2d and in particular to the portion to be analysed and wherein the optical pointer 25 and therefore the reference 2e is moved according to said ideal position.

The ideal position of the medical instrument 1c identifies the point and preferably the orientation that the medical instrument 1c should ideally assume with respect to the radiological image (therefore to the patient) before starting the intervention. It can thus only identify the position of the medical instrument 1c with respect to the patient but not how to insert it into the patient and/or perform the operation itself.

The ideal location includes a set of coordinates. In detail, it comprises a first set of coordinates (hereinafter referred to as the ideal target) defining the point in the radiological image where the medical instrument 1c and in particular the functional end 1d is to be placed; and a second set of coordinates (hereinafter referred to as ideal inclination) defining the inclination/orientation of the medical instrument 1c when it has the functional end 1d in the ideal target.

The ideal target is substantially external to the patient and in detail on the skin or other visible surface of the patient, or rather on the perimeter sector.

The planning phase 180 can be subsequent to the reconstruction phase 160 and in detail of display 170.

It can be prior to and/or contemporaneous with the video displaying phase 170. The ideal position identifies the position in which the instrument 1c must be to start and then perform an operation correctly.

The planning step 180 defines the ideal contact position of a medical instrument 1c with the patient before performing the operation.

The planning phase 180 may comprise a selection sub-phase **181** wherein the ideal position of the medical instrument 1c is defined by identifying at least ideal target and ideal inclination on the radiological image and a pointing sub-phase **182** of the optical pointer 25 and therefore of reference 2e.

In detail, in the selection sub-phase 181 an intervention target and an intervention trajectory are selected on the radiological image. This selection can be automatic, or rather performed only by the control unit, for example following an input given by the operator, or manual, or rather by the operator selecting the intervention target and insertion trajectory on the radiological image.

The intervention objective identifies a point on the radiological image, while the intervention trajectory identifies a direction along which to move/insert and then position the medical instrument 1c.

At this point the ideal position of the instrument 1c is suitably defined as a function of objective and trajectory of intervention by the control unit alone.

The ideal target depends on the intervention objective and in some cases the intervention trajectory. In detail, if the intervention objective is a point in the perimeter sector of the radiological image (for example a point on the patient's skin), the intervention objective is the ideal target. Alternatively, if the target is a point in the sector of interest of the radiological image (therefore being a point inside the patient such as an organ or a bone), the ideal target is identified as the intersection between the perimeter sector and the trajectory of the intervention. for said goal.

The ideal inclination can be a function of the intervention trajectory. In particular, it is substantially parallel and preferably almost coincident with the insertion trajectory. Once the ideal position has been identified, the pointing sub-phase 182 occurs in which the gantry 2 can move the optical pointer 25 by arranging the aiming axis 2f incident to the ideal target and therefore the optical reference 2e in correspondence with the ideal target.

In the pointing sub-phase 182 the optical pointer 25 is moved by rotating the rotor 23 around the rotation axis 2c and/or by translating the gantry 2 along the translation axis 4a so as to have the optical reference 2e in correspondence with the target ideal of the selection sub-phase 181.

It is highlighted how this operation, as well as others even if not specified, is possible thanks to the association of each virtual coordinate of a point in the radiological image to a real coordinate and therefore to virtual coordinates of the ideal target to the real coordinates of the same ideal target and of the virtual coordinates of the ideal inclination to the real coordinates of the same ideal inclination.

The acquisition method 100 can comprise an orientation phase **190** of a medical instrument 1c with respect to the scanning area 2d and in particular to the portion to be analysed.

The orientation phase 190 can be subsequent to the planning phase 180 and in detail to the pointing sub-phase 182. It can be prior to and/or contemporaneous with the video displaying phase 170.

In the orientation phase 190 the control unit compares the real position of the medical instrument 1c, or rather the position according to an optical acquisition, with respect to the ideal position and in detail to the ideal target and the ideal inclination. The orientation phase 190 can comprise a positioning sub-phase **191** of the medical instrument 1c in the scanning area 2d (in particular in contact with the portion to be analysed) suitably in correspondence with the optical reference 2e preferably with the proximal functional end 1d and in detail in contact with the optical reference 2.

In the positioning sub-phase 191 and therefore in the orientation phase 190, only the positioning of the medical instrument 1c can be provided. The insertion of the instrument 1c and/or the execution of the intervention can thus be excluded from them.

The orientation phase 190 can comprise a shooting sub-phase **192** wherein an optical acquisition of the position of the medical instrument 1c 1c and suitably of the optical reference 2e is performed.

In this shooting sub-phase 192 the acquirer 29 takes up the medical instrument 1c (in detail at least the functional end 1d) and suitably the optical reference 2e.

The shooting sub-phase 192 can be performed when, thanks to the optical acquisition, the functional end 1d is identified in correspondence with the optical reference 2a.

The orientation phase 190 can comprise a verification sub-phase **193** wherein a real position of the medical instrument 1c is defined and then checked whether it is in the ideal position with respect to the scanning area 2d and in particular to the portion to be analysed and/or to the optical reference 2e.

In the verification sub-phase 193 a real position of the medical instrument 1c is defined as a function of said optical acquisition performed in the sub-phase 192 (suitably to the tools database) and therefore the real position is compared with the ideal position and therefore with an ideal inclination and ideal target.

The real position can be a function of said optical acquisition of the medical instrument 1c and suitably of the optical reference 2e and in some cases of the instruments database.

In this sub-phase 193 the control unit identifies, with respect to the scanning area 2d and in particular and to the portion to be analysed (in detail at the optical reference 2e), the real position by defining the real target and preferably the real inclination suitably with respect to an absolute and/or specific reference of the device.

The real target identifies the point where the medical instrument 1c actually is (in particular the functional end 1d) with respect to the scanning area 2d and in particular to the portion to be analysed.

The orientation phase 190 may comprise a signalling sub-phase **194,** for example through the interface 7, whether the medical instrument 1c is substantially or not in said ideal position.

In detail, the control unit compares the real position with the ideal one. If the difference between these positions is substantially lower than an acceptability threshold in the signalling sub-phase 194, the control unit signals the correctness of the position of the medical instrument 1c.

It is pointed out that, as anticipated above, the phases 190, 180 and/or 170 (and in detail 150 and 160) can be performed simultaneously so as to allow the operator to follow the movement of the medical instrument 1c.

The radiological imaging device 1 and the method 100 according to the invention achieve important advantages.

In fact, the radiological imaging device 1 and the method 100, thanks to the use and in particular to the particular positioning of the optical pointer 25, require a particularly rapid and precise targeting.

Another important advantage is represented by the fact that the radiological imaging device 1 and the procedure 100 allow the execution of a wide typology of radiological acquisitions thanks above all to the presence of the connector 26 which allows to use an additional source 27.

Another advantage is that the radiological imaging device 1, compared to known radiological imaging devices, has a relatively simple structure and therefore has a low purchase and maintenance cost and is easy to manufacture and use.

A not secondary advantage is given by the guides 4 and in detail by the particular structure 3 which allows the gantry 2 to translate and therefore to perform a multi-stack radiological acquisition without interfering with the operator. In fact, since the guide 4 is external to the vertical projection of the scanning area 2d and in particular of the gantry, it defines a free access area through which the patient can easily position himself near the scanning area 2d and allow the gantry to be moved along the translation axis 4a without interfering with the patient himself and/or components of the device 1.

Furthermore, the use of the particular guides 4 and of a supporting structure able to distance the gantry 2 from the supporting surface 1b allows, for example, to place a radiological bed or other radiological support extremely close to the device 1. In particular, said spacing between the support surface 1b and gantry 2 allows any support of the radiological support to be inserted between the support surface 1b and gantry 2 allowing a practical juxtaposition of the patient placed in the scanning area 2d and in detail on said support surface of the radiologic support.

The invention is susceptible of variants falling within the scope of the inventive concept defined by the claims.

For example, the camera 53 may comprise a hinge configured to allow the camera itself to rotate with respect to the device 1 (in detail the second column 32) by varying the inclination of the shooting axis 53a with respect to the longitudinal axis 1a and/or to the support surface 1b.

Said hinge can be motorized.

This inclination variation can be controlled automatically (or rather from the control unit) and/or manually by the operator, for example through the interface.

In another example the additional emission apparatus 271, when the additional source 27 is connected to the connector 26, can be arranged in proximity to the rear face of the gantry 2 and therefore on the opposite side to the source 21 with respect to the gantry itself.

In another example, in the reconstruction phase 160, the optical image can be reconstructed on the basis of the one or more optical acquisitions performed in the acquisition phase 150. Preferably in the reconstruction phase 160 a composite image of the optical image and the radiological image.

The composite image can be identified by an image, for example three-dimensional, wherein the optical image represents the external profile and the radiological image identifies the internal structure. The operator can then decide whether to view the external profile to identify an area of interest (easily identifiable on the patient) and then view the underlying internal structure called an area of interest.

It should be noted that in the video displaying phase 170 the medical instrument 1c and suitably the optical and/or composite image can be exposed.

The association between optical image and radiological image can be performed by exploiting, for example, the acquisitions database and in particular the association between each single radiological acquisition and the point/corner where said radiological acquisition was performed.

The optical image and in particular the composite image can be used in the planning phase 180. In this case in the planning phase 180 the operator can select the target on the external profile.

In some cases, the medical instrument 1c, to perform the identification of its position and/or orientation, can comprise one or more additional optical references **1e** (for example a coloured block) which can be acquired by the acquirer 29. Preferably one or more additional optical references 1e are available in a distal position from each other and in particular from the optical reference 2e (in detail from the functional end 1d of the instrument).

In this case in the orientation phase 190 (in detail in the shooting sub-phase 192) the acquirer 29 also acquires the one or more additional optical references 1e. Therefore, in the verification sub-step 193, the real position of the medical instrument 1c with respect to the scanning area 2d is also determined as a function of the one or more additional optical references 1e.

It should be noted that one or more of the aforementioned examples can be simultaneously provided in the radiological imaging device 1 and/or integrated with any of the characteristics of the device 1 described above.

## Claims

1. A radiological imaging device (1) configured to rest on a support surface (1b), defining a longitudinal axis (1a) and comprising:
- a gantry (2) configured to perform the radiological acquisition of at least a portion (5) of the patient to be analysed; said gantry comprising
- a source (21) configured to define an acquisition beam and an acquisition axis (2a);
- a detector (22) configured to be etched by said acquisition beam after it has passed through said portion to be analysed;
- a rotor (23) supporting said source (21) and said detector (22) and defining a scanning zone (2d) in which at least said portion to be analysed is available;
- a stator (24) supporting said rotor (23) and configured to rotate said rotor (23) around said scanning zone (2d) defining a rotation axis (2c) substantially not perpendicular to said support surface (1b);
- a support structure (3) of said gantry (2) and resting on said support surface (1b);
- at least one guide (4) defining a translation axis (4a) of said gantry (2) with respect to said support structure (3) substantially parallel to said rotation axis (2c);
and **characterized by**
- said guide (4) being external to the vertical projection of said scanning zone (2d) so as to define an access area to said free scanning zone (2d) through which said patient can position himself in proximity to said scanning zone (2d) and allowing said gantry (2) to be translated along said translation axis (4a) by performing a multi-stack radiological acquisition.

2. The radiological imaging device (1) according to claim 1, wherein:
- said support structure (3) comprises a first column (31) and a second column (32) arranged on opposite sides with respect to said gantry (2);
- said at least one guide (4) comprises a first guide (4) binding said first column (31) to said gantry (2) and a second guide (4) binding said second column (32) to said gantry (2); and
- said guides (4) are motorized and actuated synchronously.

3. The radiological imaging device (1) according to at least the preceding claim, comprising:
- a guide apparatus (4) configured to control the movement of said radiological imaging device (1) along said support surface (1b); and
- a conduction apparatus (5) of said radiological imaging device (1);
wherein
- said conduction apparatus (5) comprises a shooting block (51) defining a shooting axis (51a) substantially transversal to said longitudinal axis,
- a control means (52) is provided for the displacement of said radiological imaging device (1) along said support surface (1b);
- a screen (54) is provided configured to display the shooting of said shooting block (51);
- said shooting block (51) is integral with said second column (32); and
- said control means (52) and said screen (54) are integral with said first column (31).

## Patentansprüche

1. Radiologische Bildgebungsvorrichtung (1), dazu konfiguriert, auf einer Stützoberfläche (1b) zu ruhen, eine Längsachse (1a) definierend und umfassend:
- eine Gantry (2), die dazu konfiguriert ist, die radiologische Erfassung mindestens eines Teils (5) des zu analysierenden Patienten durchzuführen; wobei die Gantry Folgendes umfasst:
- eine Quelle (21), die dazu konfiguriert ist, einen Erfassungsstrahl und eine Erfassungsachse (2a) zu definieren;
- einen Detektor (22), der dazu konfiguriert ist, durch den Erfassungsstrahl geätzt zu werden, nachdem er den zu analysierenden Teil durchlaufen hat;
- einen Rotor (23), der die Quelle (21) und den Detektor (22) stützt und eine Abtastzone (2d) definiert, in der mindestens der zu analysierende Teil vorhanden ist;
- einen Stator (24), der den Rotor (23) stützt und dazu konfiguriert ist, den Rotor (23) um die Abtastzone (2d) herum zu drehen, die eine Drehachse (2c) definiert, die im Wesentlichen nicht senkrecht zu der Stützoberfläche (1b) ist;
- eine Stützstruktur (3) der Gantry (2) und die auf der Stützoberfläche (1b) ruht;
- mindestens eine Führung (4), die eine Translationsachse (4a) der Gantry (2) in Bezug auf die Stützstruktur (3) im Wesentlichen parallel zu der Drehachse (2c) definiert;
und **gekennzeichnet dadurch, dass**
- die Führung (4) außerhalb der vertikalen Projektion der Abtastzone (2d) liegt, um einen Zugangsbereich zu der freien Abtastzone (2d) zu definieren, durch den sich der Patient in der Nähe der Abtastzone (2d) positionieren kann, und um zu ermöglichen, dass die Gantry (2) entlang der Translationsachse (4a) durch Durchführen einer radiologischen Mehrstapelerfassung verschoben wird.

2. Radiologische Bildgebungsvorrichtung (1) nach Anspruch 1, wobei:
- die Stützstruktur (3) eine erste Säule (31) und eine zweite Säule (32) umfasst, die auf gegenüberliegenden Seiten in Bezug auf die Gantry (2) angeordnet sind;
- die mindestens eine Führung (4) eine erste Führung (4), die die erste Säule (31) an die Gantry (2) bindet, und eine zweite Führung (4), die die zweite Säule (32) an die Gantry (2) bindet, umfasst; und
- die Führungen (4) motorisiert sind und synchron betätigt werden.

3. Radiologische Bildgebungsvorrichtung (1) nach mindestens dem vorhergehenden Anspruch, umfassend:
- eine Führungseinrichtung (4), die dazu konfiguriert ist, die Bewegung der radiologischen Bildgebungsvorrichtung (1) entlang der Stützoberfläche (1b) zu steuern; und
- eine Leitungseinrichtung (5) der radiologischen Bildgebungsvorrichtung (1);
wobei
- die Leitungsvorrichtung (5) einen Aufnahmeblock (51) umfasst, der eine Aufnahmeachse (51a) im Wesentlichen quer zu der Längsachse definiert,
- ein Steuerungsmittel (52) zum Verlagern der radiologischen Bildgebungsvorrichtung (1) entlang der Stützoberfläche (1b) bereitgestellt ist;
- ein Bildschirm (54) bereitgestellt ist, der dazu konfiguriert ist, das Aufnehmen des Aufnahmeblocks (51) anzuzeigen;
- der Aufnahmeblock (51) einstückig mit der zweiten Säule (32) ist; und
- das Steuerungsmittel (52) und der Bildschirm (54) einstückig mit der ersten Säule (31) sind.

## Revendications

1. Dispositif d'imagerie radiologique (1) conçu pour reposer sur une surface de support (1b), définissant un axe longitudinal (1a) et comprenant :
- un portique (2) configuré pour effectuer l'acquisition radiologique d'au moins une partie (5) du patient à analyser ; ledit portique comprenant
- une source (21) configurée pour définir un faisceau d'acquisition et un axe d'acquisition (2a) ;
- un détecteur (22) configuré pour être gravé par ledit faisceau d'acquisition après que celui-ci a traversé ladite partie à analyser ;
- un rotor (23) supportant ladite source (21) et ledit détecteur (22) et définissant une zone de balayage (2d) dans laquelle au moins ladite partie à analyser est disponible ;
- un stator (24) supportant ledit rotor (23) et configuré pour faire tourner ledit rotor (23) autour de ladite zone de balayage (2d) définissant un axe de rotation (2c) sensiblement non perpendiculaire à ladite surface de support (1b) ;
- une structure de support (3) dudit portique (2) et reposant sur ladite surface de support (1b) ;
- au moins un guide (4) définissant un axe de translation (4a) dudit portique (2) par rapport à ladite structure de support (3) sensiblement parallèle audit axe de rotation (2c) ;
et **caractérisé par :**
- ledit guide (4) étant externe à la projection verticale de ladite zone de balayage (2d) de manière à définir une zone d'accès à ladite zone de balayage libre (2d) à travers laquelle ledit patient peut se positionner à proximité de ladite zone de balayage (2d) et permettant audit portique (2) d'être translaté le long dudit axe de translation (4a) en effectuant une acquisition radiologique multi-couches.

2. Dispositif d'imagerie radiologique (1) selon la revendication 1, dans lequel :
- ladite structure de support (3) comprend une première colonne (31) et une seconde colonne (32) disposées sur des côtés opposés par rapport audit portique (2) ;
- ledit au moins un guide (4) comprend un premier guide (4) reliant ladite première colonne (31) audit portique (2) et un deuxième guide (4) reliant ladite seconde colonne (32) audit portique (2) ; et
- lesdits guides (4) sont motorisés et actionnés de manière synchrone.

3. Dispositif d'imagerie radiologique (1) selon au moins la revendication précédente, comprenant :
- un appareil de guidage (4) conçu pour commander le mouvement dudit dispositif d'imagerie radiologique (1) le long de ladite surface de support (1b) ; et
- un appareil de conduction (5) dudit dispositif d'imagerie radiologique (1) ;
dans lequel
- ledit appareil de conduction (5) comprend un bloc de prise de vue (51) définissant un axe de prise de vue (51a) sensiblement transversal audit axe longitudinal,
- des moyens de commande (52) sont prévus pour le déplacement dudit dispositif d'imagerie radiologique (1) le long de ladite surface de support (1b) ;
- un écran (54) est prévu configuré pour afficher la prise de vue dudit bloc de prise de vue (51) ;
- ledit bloc de prise de vue (51) est solidaire de ladite seconde colonne (32) ; et
- lesdits moyens de commande (52) et ledit écran (54) sont solidaires de ladite première colonne (31).
